# EUROPEAN PATENT APPLICATION

(11) **EP 4 454 484 A1**
(43) Date of publication of application: **30.10.2024**
(21) Application number: 22911388.1
(22) Date of filing: 23.12.2022
(51) Int. Cl.: A23L 33/105, A23L 19/00, A61K 8/9794, A61K 36/888, A61P 17/00, A61P 25/28, A61Q 19/00

(54) **WATER-SOLUBLE KONJAC CORM EXTRACT AND METHOD FOR PRODUCING SAME**

(30) Priority: 24.12.2021 JP 2021215581
(71) Applicant: Yukiguni Aguri Co., Ltd., Numata-shi, Gunma 378-0004 (JP)
(72) Inventor: MUROI, Fumihiro, Numata-shi, Gunma 378-0004 (JP)
(74) Representative: Schulz Junghans Patentanwälte PartGmbB
(86) International application number: PCT/JP2022/047537
(87) International publication number: WO 2023/120686

(57) **Abstract**

The objective of the present invention is to provide a water-soluble konjac potato extract with a simpler method. There is provided a water-soluble konjac potato extract comprising sterylglucoside, wherein the content of fatty acid ester is equal to or less than 500 mg/100 g, the content of phytosterol is equal to or less than 5,000 mg/100 g, and the content of glucosylceramide is equal to or more than 5,000 mg/100 g, obtained by separating and removing fatty acid ester and phytosterol from a konjac potato extract extracted with an organic solvent.

## Description

### Technical Field

The present invention relates to a water-soluble konjac potato extract and a method of producing a water-soluble konjac potato extract.

### Background Art

Konnyaku imo (konjac potato) is a traditional Japanese agricultural product and has attracted much attention because of high contents of dietary fiber and other useful components. In the course of producing konjac flour, which is used as a raw material for ita-konjac or shirataki, a large amount of konjac flying-out flour is produced as a byproduct. Konjac flying-out flour contains many useful components, especially a very high content of sphingoglycolipid represented by glucosylceramide. Some methods of producing sphingoglycolipid and other components from konjac flying-out flour have been known (for example, Patent Document 1, Patent Document 2, and Patent Document 3).

Sphingoglycolipid is digested and assimilated in the body through oral intake, and has the function of improving skin moisturizing property. Sphingoglycolipid is marketed as one of Foods with Functional Claims. Sphingoglycolipid is also known to improve skin moisturizing property and skin abnormalities such as dry skin, rough skin, and atopic dermatitis when applied directly to the skin. Recently, a new functionality of sphingoglycolipid has been discovered as Alzheimer's preventive agent (for example, Patent Document 4). Sphingoglycolipid has attracted attention not only in the food field but also in the pharmaceutical and cosmetic fields.

In addition to konjac potato, sphingoglycolipid is found in a variety of plants, including wheat, rice, corn, peach and pineapple. So far, sphingoglycolipid derived from such plants has been marketed for food and cosmetic applications. However, the plant extract containing sphingoglycolipid is completely insoluble in water as it stands. Therefore, the method of making the plant extract containing sphingoglycolipid into a water-soluble composition by utilizing emulsifiers such as polyglycerol fatty acid ester is known (for example, Patent Document 5). The konjac potato extract containing sphingoglycolipid extracted from konjac potato also precipitates without hardly dissolving or suspending in water as it stands when extracted with an organic solvent. In order to stably dissolve the konjac potato extract in water, emulsifiers such as polyglycerol fatty acid ester, sucrose fatty acid ester, and enzyme-degraded lecithin have been employed to solubilize the konjac potato extract and to make it possible to contain the resultant in various drink, cosmetic and pharmaceutical products.

On the other hand, the following methods of obtaining the extract containing sphingoglycolipid from konjac potato are known; the method of dissolving a crude extract of konjac potato in methanol to obtain a solution followed by introducing the solution into column chromatography (for example, Patent Document 6), the method of dissolving a crude extract of konjac potato in petroleum ether to obtain a solution followed by introducing the solution into column chromatography (for example, Patent Documents 7 to 11), and the method of subjecting a crude extract of konjac potato to solvent extraction using alkaline methanol and chloroform (for example, Patent Document 12).

### Citation List

### Patent Document

[Patent Document 1] JP 3992425 B
[Patent Document 2] JP 3650587 B
[Patent Document 3] JP 4753476 B
[Patent Document 4] JP 2019-078005 A
[Patent Document 5] JP 3395444 B
[Patent Document 6] JP 2003-221592 A
[Patent Document 7] CN 102190689 A
[Patent Document 8] CN 102675139 A
[Patent Document 9] CN 108623492 A
[Patent Document 10] CN 102351730 A
[Patent Document 11] CN 108947864 A
[Patent Document 12] JP 2011-195550 A

### Summary of the Invention

### Problems to be Solved by the Invention

As mentioned above, the method described in Patent Document 5 uses an emulsifier in combination with other components to solubilize the konjac potato extract. However, depending on the emulsifier used or the combination of emulsifier and other components, the resulting aqueous composition may have poor emulsion stability. Therefore, the desired method is to allow the aqueous composition containing the konjac potato extract to have improved solubility without adding any emulsifier.

On the other hand, each water solubility of the konjac potato extracts obtained by the methods described in Patent Documents 6 to 12 has not been clarified. In addition, the methods described in Patent Documents 7 to 12 use petroleum ether (mainly pentane) and chloroform, which have not been permitted as extraction solvents under the production standards of the Japan's Specifications and Standards for Food Additives.

It is an objective of the present invention to provide a konjac potato extract which contains many useful components including sphingoglycolipid and can be used in producing aqueous compositions such as food, cosmetic and pharmaceutical products as a cosmetic ingredient, health food ingredient or pharmaceutical ingredient; and a production method thereof.

### Means for Solving the Problems

The present inventor found that the konjac potato extract extracted from konjac flying-out flour with the use of ethanol is insoluble in water. The present inventor conceived of the idea that the selective removal of components other than the useful components including sphingoglycolipid may enable a konjac potato extract to be soluble in water. Therefore, the present inventor considered a method to leave the useful components in the konjac potato extract while removing the components that prevent the konjac potato extract from being soluble in water.

As a result, the present inventor found out that the reduction of neutral lipids such as phytosterol and fatty acid ester contained in the konjac potato extract could improve the poor water solubility of konjac potato extract so that the konjac potato extract could be easily dissolved in water. As such, the present invention has been completed on the basis of the findings and successful examples that were found or obtained by the present inventor.

Therefore, each embodiment according to one aspect of the present invention is provided as follows:
(1) A water-soluble konjac potato extract, having a content of fatty acid ester of 1,000 mg/100 g or less.
(2) The water-soluble konjac potato extract according to (1), having a content of phytosterol of 5,000 mg/100 g or less.
(3) The water-soluble konjac potato extract according to (1) or (2), having a content of glucosylceramide of 5,000 mg/100 g or more.
(4) The water-soluble konjac potato extract according to any one of (1) to (3), having a content of sterylglucoside of 500 mg/100 g or more.
(5) A food, cosmetic or pharmaceutical product comprising the water-soluble konjac potato extract according to any one of (1) to (4).
(6) A method of producing a water-soluble konjac potato extract, comprising
   an extraction step of extracting a konjac potato extract from konjac potato flour using an organic solvent, and
   a separation step of separating fatty acid ester and phytosterol from the konjac potato extract by column chromatography to render a content of fatty acid ester 1,000 mg/100 g or less and a content of phytosterol 5,000 mg/100 g or less.
(7) A method of producing a water-soluble konjac potato extract, comprising
   an extraction step of extracting a konjac potato extract from konjac potato flour using an organic solvent, and
   a removal step of removing fatty acid ester and phytosterol from the konjac potato extract using other organic solvent from the organic solvent used in the extraction step to render a content of fatty acid ester 1,000 mg/100 g or less and a content of phytosterol 5,000 mg/100 g or less.

Each embodiment according to another aspect of the present invention is provided as follows:
[1] A water-soluble konjac potato extract comprising sterylglucoside, with a content of fatty acid ester of equal to or less than 500 mg/100 g, a content of phytosterol of equal to or less than 5,000 mg/100 g, and a content of glucosylceramide of equal to or more than 5,000 mg/100 g.
[2] The water-soluble konjac potato extract according to [1], wherein the content of sterylglucoside is equal to or more than 500 mg/100 g.
[3] A food, cosmetic or pharmaceutical product comprising the water-soluble konjac potato extract according to [1].
[4] A method of producing a water-soluble konjac potato extract, comprising
   an extraction step of extracting a konjac potato extract from konjac potato flour using a first organic solvent, and
   a removal step of removing fatty acid ester and phytosterol from the konjac potato extract using a second organic solvent to obtain a water-soluble konjac potato extract,
   wherein the water-soluble konjac potato extract comprises sterylglucoside, with a content of fatty acid ester of equal to or less than 500 mg/100 g, a content of phytosterol of equal to or less than 5,000 mg/100 g, and a content of glucosylceramide of equal to or more than 5,000 mg/100 g, and
   the first organic solvent and the second organic solvent are different from each other and from petroleum ether and chloroform.
[5] The method according to [4], wherein the first organic solvent is ethanol.
[6] The method according to [4], wherein the first organic solvent is acetone.

### Effects of the Invention

The effects of the present invention are as follows:
(1) According to the present invention, the fatty acid ester content of 1,000 mg/100 g or less can render the konjac potato extract soluble in water.
(2) According to the present invention, the phytosterol content of 5,000 mg/100 g or less can render the konjac potato extract soluble in water.
(3) According to the present invention, the glucosylceramide content of 5,000 mg/100 g or more can produce a water-soluble konjac potato extract containing useful components.
(4) According to the present invention, the sterylglucoside content of 500 mg/100 g or more can produce a water-soluble konjac potato extract containing useful components.
(5) According to the present invention, the extract can be used in a food, cosmetic and pharmaceutical product without the use of emulsifiers.
(6) According to the present invention, there is provided a simple method of producing a water-soluble konjac potato extract with water-solubility, including reducing the fatty acid ester content in a konjac potato extract to 1,000 mg/100 g or less and the phytosterol content to 5,000 mg/100 g or less by column chromatography.
(7) According to the present invention, there is provided a simple method of producing a water-soluble konjac potato extract with water-solubility, including reducing the fatty acid ester content in a konjac potato extract to 1,000 mg/100 g or less and the phytosterol content to 5,000 mg/100 g or less by the removal step using a different organic solvent from that used in the extraction step.

The water-soluble konjac potato extract according to one embodiment of the present invention can contain useful components such as sphingoglycolipid in a large amount and is available to a cosmetic ingredient, health food material and pharmaceutical material, which can be utilized to produce a food, cosmetic and pharmaceutical product that is a liquid composition containing glucosylceramide derived from konjac.

### Brief Description of Drawing

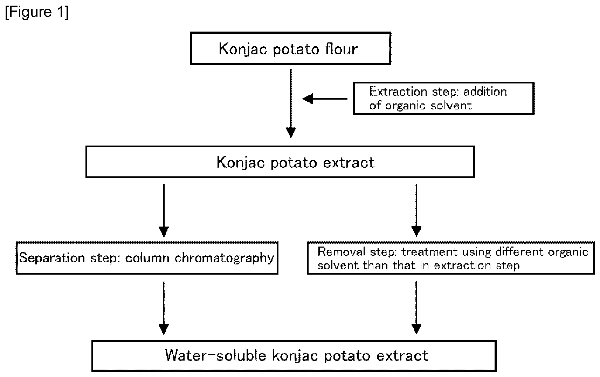

Figure 1 is a flow diagram representing a method of producing a water-soluble konjac potato extract.
Figure 2 shows the results of evaluating the solubility of test samples (a) to (c) in water, as described in Examples below.

### Description of Embodiments

One aspect of the present invention is a konjac potato extract extracted with an organic solvent and with improved water solubility. In the following, the embodiments of the konjac potato extract will be described in detail. However, the following embodiments are examples to illustrate the present invention, and the present invention is not limited only to the embodiments.

### 1. Raw materials of water-soluble konjac potato extract

The raw material konjac potato used in the embodiment may be konjac potato as it stands, or may be konjac potato processed by drying, mashing, crushing, heating or other operations. Konjac potato may be konjac refined flour, konjac fine flour, konjac medium flour, konjac rough flour, konjac flying-out flour or other konjac flour, or konjac processed and sold commercially for food use. Among them, preferred examples include konjac flying-out flour, which is generated in large quantities as a byproduct in producing konjac refined flour, konjac fine flour and konjac medium flour, is discarded after the production, and is available at low cost.

As the organic solvent (first organic solvent) used for extraction in the embodiment, any organic solvent can be used as long as it can dissolve glucosylceramide and sterylglucoside and does not react with the useful components in konjac potato during extraction, thereby not impairing the solution to the problems of the present invention. The organic solvent may be used either individually or as a mixture of two or more of organic solvents. Examples of the first organic solvent include monovalent primary alcohols such as methanol, ethanol and isopropanol; other alcohols such as 2-butanol, ethylene glycol and propylene glycol; oxygen-containing polar organic solvents such as acetone, dimethyl sulfoxide, dioxane, tetrahydrofuran and diethyl ether; nitrogen-containing polar organic solvents such as dimethylformamide and pyridine; halogen-containing polar organic solvents such as dichloromethane, chloroform and trichloroethylene; and nonpolar or low-polar organic solvents such as hexane and isooctane. The organic solvent may be mixed with water in some cases. Furthermore, the organic solvent may be a high-pressure fluid such as carbon dioxide, ethane and trifluoromethane in a high-pressure liquefied or supercritical state. Among them, ethanol is preferred when the konjac potato extract is used as a food product or food additive, and acetone, hexane, water or carbon dioxide in a supercritical or other state may be mixed as needed. In this case, the content of ethanol in the first organic solvent used for extraction is preferably 70% or more, more preferably 80% or more, still more preferably 90% or more, even still more preferably 100%. For example, the konjac potato extract obtained by using ethanol as the first organic solvent may be called a konjac potato ethanol extract.

### 2. Production of water-soluble konjac potato extract

In producing the water-soluble konjac potato extract according to this embodiment, the extraction step is carried out by adding the organic solvent to konjac potato to extract the useful components contained in konjac potato. The amount of organic solvent used for extraction may be about 1 to 30 times, preferably 1 to 20 times, more preferably 1 to 10 times relative to the amount of the raw material konjac potato.

The extraction temperature depends on the boiling point of the organic solvent used, but is preferably 0°C to 80°C, more preferably about room temperature to 60°C. The extraction time is preferably 1 hour to 48 hours, more preferably 2 hours to 20 hours.

The extraction method may be, but is not limited to, a batch operation in which konjac potato is mixed with the organic solvent in a stirring tank only once. Afresh solvent may be added again to the residue after extraction and the extraction operation may be repeated, or the organic solvent may be repeatedly brought into contact with the raw material for extraction. In other words, either batch, semi-continuous, or counter-current multi-stage contact operation may be employed as the extraction operation. Known extraction methods such as soxhlet extraction may also be used.

Next, the extraction residue is separated and removed from the extract solution obtained in the extraction step. The separation method is not preferably limited. Examples of the method include known methods such as suction filtration, filter press, cylinder press, decanter, centrifugal separator and filtration centrifugation.

The extract solution thus obtained is forwarded to the concentration step. The concentration method is not preferably limited. Examples of the method include methods by removing the solvent by a decompression concentration device such as an evaporator or a centrifugal thin-film vacuum evaporator, or by heating to concentrate the extract solution. When supercritical fluid is used as the organic solvent for extraction, the solvent may be removed by simply releasing pressure after extraction, so the concentration step may be omitted.

In this embodiment, phytosterol is reduced to 5,000 mg/100 g or less, preferably 4,000 mg/100 g or less, more preferably 3,500 mg/100 g or less; and/or fatty acid ester is reduced to 1,000 mg/100 g or less, preferably 500 mg/100 g or less, more preferably 50 mg/100 g or less, so that the konjac potato extract can be easily dissolved in water. Phytosterol is sterols represented by sitosterol, campesterol, brassicasterol, stigmasterol and sitostanol, and includes phytosterol in which fatty acids are bound to sterols. Furthermore, fatty acid ester is esters in which functional groups such as methyl, ethyl and propyl groups are bound to carboxyl groups of C₁₆ to C₂₀ saturated and/or unsaturated fatty acids. Phytosterol and fatty acid ester are determined by the methods described in Examples below.

On the other hand, the water-soluble konjac potato extract may preferably contain the useful components glucosylceramide and sterylglucoside, thereby being used for food, cosmetic and pharmaceutical applications. The content of glucosylceramide is preferably 5,000 mg/100 g or more, more preferably 5,000 mg/100 g to 50,000 mg/100 g, still more preferably 10,000 mg/100 g to 40,000 mg/100 g, even still more preferably 15,000 mg/100 g to 30,000 mg/100 g or 20,000 mg/100 g to 30,000 mg/100 g relative to the total amount (100 g) of water-soluble konjac potato extract; and/or the content of sterylglucoside is preferably 500 mg/100 g or more, more preferably 500 mg/100 g to 5,000 mg/100 g, still more preferably 1,000 mg/100 g to 4,000 mg/100, even still more preferably 1,500 mg/100 g to 3,500 mg/100 g or 2,000 mg/100 g to 3,500 mg/100 g relative to the total amount (100 g) of water-soluble konjac potato extract.

Glucosylceramide and sterylglucoside are insoluble in water and rather liposoluble. However, konjac potato contains phosphatidylcholine and other phospholipids, which act as emulsifiers and allow part of glucosylceramide or sterylglucoside to dissolve in water. However, the konjac potato extract contains fatty acid ester and phytosterol at a certain concentration in addition to glucosylceramide and sterylglucoside. It is assumed that they would inhibit the emulsifying action as phospholipids and prevent glucosylceramide and sterylglucoside in the konjac potato extract from dissolving in water. The water-soluble konjac extract according to one embodiment of the present invention has the enhanced contents of glucosylceramide and sterylglucoside while the reduced contents of fatty acid ester and phytosterol relative to the amount of the konjac potato extract, thereby being a water-soluble konjac extract.

When the water-soluble konjac potato extract is considered for food applications, the method of producing the water-soluble konjac potato extract preferably employs as the first and second organic solvents listed in the production standards of the Japan's Specifications and Standards for Food Additives (e.g., acetone, ethanol, glycerin, ethyl acetate, methyl acetate, diethyl ether, cyclohexane, dichloromethane, 1,1,2-trichloroethene, edible oil, 1,1,1,2-tetrafluoroethane, 1-butanol, 2-butanol, 2-butanone, 1-propanol, 2-propanol, propylene glycol, hexane, methanol) but preferably do not employ petroleum ether such as pentane and chloroform. The water-soluble konjac potato extract is preferably substantially free from petroleum ether and chloroform.

The method of producing the water-soluble konjac potato extract according to one embodiment of the present invention is a method that can reduce phytosterol and fatty acid ester. The method of producing the water-soluble konjac potato extract according to one embodiment of the present invention is a method using a resin or using an organic solvent different from that used in the extraction step.

The method using a resin includes a separation step to separate phytosterol and fatty acid ester from the konjac potato extract by column chromatography. The separation step is not particularly limited as long as it is capable of separating the useful components, glucosylceramide and sterylglucoside, from phytosterol and fatty acid ester. Examples of the separation step include separation steps using silica gel, ion exchange resins, affinity resins, gel filtration, hydrophobic chromatography resins and synthetic adsorbents.

The method using an organic solvent different from that used in the extraction step includes a removal step to remove phytosterol and fatty acid ester from the konjac potato extract by using a different organic solvent (second organic solvent) than that in the extraction step. The removal step preferably employs the second organic solvent that is less likely to dissolve the useful components glucosylceramide and sterylglucoside, and more likely to dissolve phytosterol and fatty acid ester. Examples of such preferred second organic solvent include acetone, hexane, supercritical carbon dioxide and mixtures thereof, of which acetone is preferred. In this case, the content of acetone in the second organic solvent is preferably 70% or more, more preferably 80% or more, still more preferably 90% or more, even still more preferably 100%.

The method using an organic solvent different from that used in the extraction step may efficiently remove phytosterol and fatty acid ester by using an organic solvent different from that used in the extraction step or an organic solvent with a different composition from that used in the extraction step.

After the extraction step, the konjac potato extract removing the organic solvent used in the extraction step may be treated in the same manner as the extraction step in the removal step of removing phytosterol and fatty acid ester by adding a different organic solvent from that used in the extraction step. In other words, the amount of organic solvent used to remove phytosterol and fatty acid ester is preferably about 1 to 30 times, more preferably about 5 to 20 times the amount, still more preferably about 5 to 10 times relative to the amount of konjac potato extract. The dissolution in the organic solvent may be accelerated by stirring or the other operation. The extraction step and the removal step are preferably carried out in a continuous manner in order to simplify the operations and enhance the yield of water-soluble konjac potato extract. In other words, the preferred embodiment of the method is preferably to use the konjac potato extract obtained by the extraction step for the removal step without subjecting the extract to any other treatment.

The treatment temperature depends on the boiling point of the organic solvent used, but is preferably 0°C to 80°C, more preferably room temperature to 60°C.

The treatment time is, for example, 1 hour to 48 hours, preferably 2 hours to 20 hours.

The treatment in the removal step may be, but is not limited to, only one batch operation. A fresh organic solvent may be added again to the residue after the first treatment and the treatment may be repeated, or the organic solvent may be repeatedly brought into contact with the konjac potato extract. In other words, either batch, semi-continuous, or counter-current multi-stage contact operation may be employed as the operation. Known extraction methods such as soxhlet extraction may also be used.

The material (residue) treated with the organic solvent is separated and removed. The separation method is not particularly limited. Examples of the method include known methods such as suction filtration, filter press, cylinder press, decanter, centrifugal separator and filtration centrifugation.

The water-soluble konjac potato extract finally obtained may be used as it is, or after removing organic solvents used in column chromatography or other operation if necessary. The water-soluble konjac potato extract may be dissolved in the organic solvent used in the extraction step. After this treatment, the organic solvent may be removed to obtain the water-soluble konjac potato extract in solid form.

The water-soluble konjac potato extract according to one embodiment of the present invention is one obtained by the method described above in which a content of phytosterol is 5,000 mg/100 g or less; and/or a content of fatty acid ester is 1,000 mg/100 g or less. If the above contents exceed 5,000 mg/100 g and/or 1,000 mg/100 g, respectively, the konjac potato extract is not completely dissolved in water, and the insoluble portion is present and precipitates with the passage of time. In this case, the konjac potato extract is difficult to stably dissolve in water. Alternatively, in this case, once the konjac potato extract is dissolved in water, part of the water-insoluble konjac potato extract precipitates out of the solution with the passage of time. By reducing phytosterol in the konjac potato extract to 5,000 mg/100 g or less and/or by reducing fatty acid ester in the konjac potato extract to 1,000 mg/100 g or less, the water-soluble konjac potato extract may be utilized as a cosmetic, food or pharmaceutical ingredient used in a form dissolved in water.

The water-soluble konjac potato extract according to one embodiment of the present invention may be excellent when added to food and cosmetic products because the extract contains large amounts of sphingoglycolipid and sterylglucoside, which play important roles in skin moisturization and against dementia and other symptoms. Examples of the food product include health foods, health drinks, staple foods such as bread, udon, soba, and rice, confectionery such as cookies, cakes, jellies, puddings, candies, chewing gum, and yogurt, soft drinks, alcoholic beverages, coffee, tea, and milk. Examples of the cosmetic product include lotions, milky lotions, moisture creams, perfumes, lip balms, lipsticks, and other products applied to the skin, hair care products, hair growth products, pomades, setting lotions, hair sprays, hair dyes, hair tonics, eyelash care products, and other products applied to the hair, face wash creams, face soap, shampoos, rinse, hair treatment, and other products used during face or hair washing as well as bath products.

### Examples

The present invention will now be described in further detail with reference to the following Examples. However, each technical feature and their combination in each embodiment is an example, and additions, omissions, substitutions, and other changes to the feature may be made as appropriate as long as the scope does not deviate from the main purpose of the present invention. The present invention is not limited by embodiments, but limited only by the scope of the claims.

The measurement devices and methods employed in the following Examples will now be described.

### (1) Quantitative methods for phytosterol and fatty acid ester

The quantities of phytosterol and fatty acid ester were determined using gas chromatography (GC). Agilent Technologies HP 6890 Series GC System was used. To detect each component, a hydrogen flame ionization detector (FID) was used. Agilent Technologies HP-5MS column (30 m long, 0.25 mm diameter, 0.25 µm film thickness) was used for the separation of phytosterol. The detector temperature was 300°C, the flow rate was 1.0 mL/min (carrier gas: He), the injector temperature was 150°C, the injection volume was 1 µL, and the split ratio was 50:1. The column oven temperature was set at a starting temperature of 250°C, increased to 300°C at 10°C/minute, and held for 10 minutes after reaching 300°C. Agilent Technologies DB-WAX column (60m long, 0.25 mm diameter, 0.25 µm film thickness) was used for the separation of fatty acid ester. The detector temperature was set at 280°C, the flow rate 1.0 mL, and the split ratio was 50:1. The column oven temperature was set at a starting temperature of 50°C, held for 1 minute, and increased to 200°C at 25°C/minute and then to 230°C at 3°C/minute, and held for 13 minutes after reaching 230°C.

As standard reagents, β-sitosterol (Tama Biochemical), campesterol (Tama Biochemical), stigmasterol (Sigma), ethyl linoleate (Sigma), ethyl palmitate (Fujifilm Wako Pure Chemical), and fatty acid methyl ester mix for qualitative use (F. A. M. E. Mix, C4-C24) (Sigma) were used. The fatty acid ethyl ester mix for qualitative use was prepared by subjecting the fatty acid methyl ester mix for qualitative use to ester exchange reaction. Phytosterol and fatty acid ester were determined using these standard reagents.

### (2) Quantitative methods for glucosylceramide and sterylglucoside

High performance liquid chromatography (HPLC) was used to quantify glucosylceramide and sterylglucoside. Shimadzu LC-20A HPLC was used, and the detector used was Shimadzu ELSD-LTIII evaporative light scattering detector. The column used was Inertsil SIL100A from GL Sciences. The solvent used was chloroform:methanol = 9:1 (volume ratio), and the determination was carried out at a flow rate of 1.0 mL/min at 37°C. Glucosylceramide derived from konjac potato (Nagara Science) and β-sitosterol-3-O-glucoside (Fujifilm Wako Pure Chemical) were used as standard reagents.

### [Reference Example 1]

One kg of konjac flying-out powder was prepared in a stirring tank, 2 L of ethanol was added thereto, and the added materials were stirred for 2 hours at room temperature. The resulting mixture was then separated into an extract solution and a residue by filtration. The extract solution was concentrated using an evaporator to obtain 10.7 g of a brown waxy concentrate (konjac potato extract). The extract was analyzed for phytosterol, fatty acid ester, glucosylceramide and sterylglucoside using the determination methods described above. The results are shown in Table 1. In addition, 1 g of the konjac potato extract was stirred in a beaker containing 1 L of 70°C warm water for 10 minutes and then left to return to room temperature. As a result, the konjac potato extract precipitated at the bottom of the beaker as a waxy mass, all of which was not stably dissolved in water. Even when methanol was used in place of ethanol, the konjac potato extract was obtained as a brown waxy concentrate. The konjac potato extract obtained in this way was not stably soluble in water.

### [Example 1]

The solution obtained by dissolving 10.0 g of the konjac potato extract obtained in Reference Example 1 in 100 mL chloroform was passed through a 300-mL volume glass column packed with silica gel. The silica gel column was then washed with three times its volume of chloroform relative to the column volume followed by one time its volume of chloroform/methanol (9/1) the column volume. Furthermore, two times its volume of chloroform/methanol (8/2) the column volume was passed through the silica gel column to elute the konjac potato extract from the silica gel column. The chloroform/methanol solvent was removed from the eluted solution using an evaporator to yield 3.2 g of a light brown blocky solid (water-soluble konjac potato extract). This water-soluble konjac potato extract was analyzed as in Reference Example 1. The results are shown in Table 1. In addition, 1 g of the water-soluble konjac potato extract was stirred in a beaker containing 1 L of 70°C warm water for 10 minutes and then left to return to room temperature. As a result, the water-soluble konjac potato extract was completely dissolved in water, and no precipitation was observed at all. Furthermore, when the resulting aqueous solution was sealed and left in an incubator at 40°C for one week, no precipitation occurred, and the extract remained completely dissolved in water.

### [Example 2]

The mixture obtained by adding 100 mL of acetone (10 times the volume) to 10.0 g of the konjac potato extract obtained in Reference Example 1 was stirred for 2 hours at room temperature, and then separated into an acetone solution and a residue by filtration. The mixture obtained by adding 100 mL of ethanol to the residue was stirred and dissolved, and then immediately filtrated to obtain an ethanol solution. The ethanol solution was subjected to solvent removal treatment using an evaporator to remove ethanol to obtain 4.6 g of a light brown blocky solid (water-soluble konjac potato extract). This water-soluble konjac potato extract was analyzed as in Reference Example 1. The results are shown in Table 1. In addition, 1 g of the water-soluble konjac potato extract was stirred in a beaker containing 1 L of 70°C warm water for 10 minutes and then left to return to room temperature. As a result, the water-soluble konjac potato extract was completely dissolved in water, and no precipitation was observed at all. Furthermore, when the resulting aqueous solution was sealed and left in an incubator at 40°C for one week, no precipitation occurred, and the extract remained completely dissolved in water.

**[Table 1]**

| | Phytosterol | Fatty acid ester | Glucosylceramide | Sterylglucoside |
|---|---|---|---|---|
| Reference Example 1 | 9,638 | 3,651 | 10,100 | 1,400 |
| Example 1 | 1,036 | 7 | 28,300 | 3,000 |
| Example 2 | 3,079 | 29 | 21,000 | 2,300 |

| | | | | |
|---|---|---|---|---|
| (Unit: mg/100 g) | | | | |

### [Example 3]

The mixture obtained by adding 100 mL of acetone (10 times the volume) to 10.0 g of the konjac potato extract obtained from konjac flying-out flour as with the method described in Reference Example 1 was stirred for 2 hours at room temperature, and then separated into an acetone solution and a residue by filtration. The mixture obtained by adding 100 mL of ethanol to the residue was stirred and dissolved to obtain an ethanol solution.

Since fatty acid ester, which prevents the konjac potato extract from dissolving in water, was extracted in the acetone solution, a certain amount of the acetone solution was combined with the ethanol solution to prepare a konjac potato extract with a fatty acid ester content of 320 mg/100 g (test sample (b)) and a konjac potato extract with a fatty acid ester content of 750 mg/100 g (test sample (c)). After the solvent was removed from the resulting konjac potato extract under reduced pressure, the solubility of the resulting light brown blocky solid in water was evaluated. The water-soluble konjac potato extract of Example 2 (the fatty acid ester content was 29 mg/100 g; test sample (a)) was also evaluated.

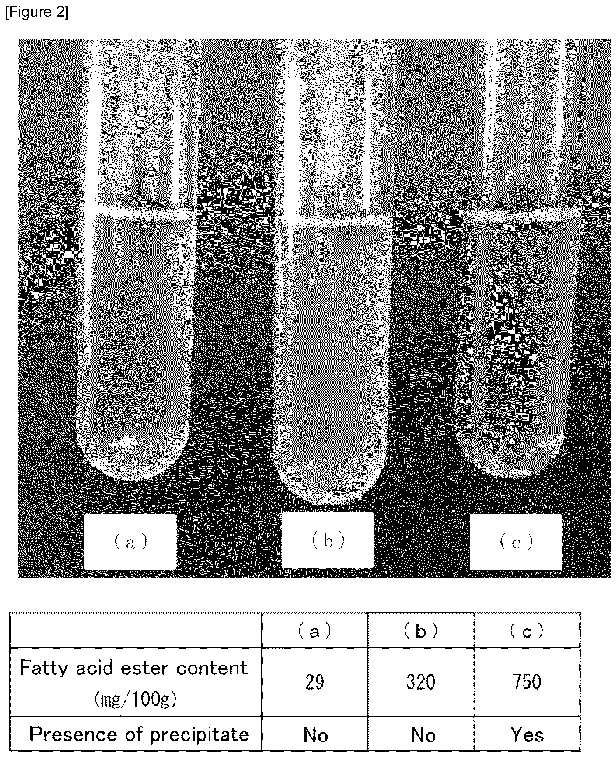

Each test sample was added and stirred to 70°C warm water to become the concentration of 1 g/1 L, the resulting mixture was left to return to room temperature. The photographic diagram of the solution appearance is shown in Figure 2. As shown in Figure 2, the test sample (a) and the test sample (b) dissolved in water and produced little precipitation, whereas the test sample (c) hardly dissolved in water and produced an agglomerated precipitate.

Therefore, it was found that the konjac potato extract in which the fatty acid ester content was less than 750 mg/100 g was a water-soluble konjac potato extract that was soluble in water.

### [Example 4]

The attempt was that a water-soluble konjac potato extract was obtained in the same manner as in Example 2, except that the amount of acetone was set at 5 times the volume (50 mL) or 20 times the volume (200 mL). As a result, even in the case where the amount of acetone was set at 5 times the volume (50 mL) or 20 times the volume (200 mL), the water-soluble konjac potato extract could be obtained as in Example 2.

### Cross-reference of related applications

The present application claims the benefit of priority to Japanese Patent Application No. 2021-215581, filed on December 24, 2021, the disclosure of which is incorporated herein by reference in its entirety.

The disclosure of all the documents described herein including Patent Documents 1 to 12 is incorporated herein by reference in its entirety.

## Claims

1. A water-soluble konjac potato extract comprising sterylglucoside, with a content of fatty acid ester of equal to or less than 500 mg/100 g, a content of phytosterol of equal to or less than 5,000 mg/100 g, and a content of glucosylceramide of equal to or more than 5,000 mg/100 g.

2. The water-soluble konjac potato extract according to claim 1, wherein the content of sterylglucoside is equal to or more than 500 mg/100 g.

3. A food, cosmetic or pharmaceutical product comprising the water-soluble konjac potato extract according to claim 1.

4. A method of producing a water-soluble konjac potato extract, comprising
an extraction step of extracting a konjac potato extract from konjac potato flour using a first organic solvent, and
a removal step of removing fatty acid ester and phytosterol from the konjac potato extract using a second organic solvent to obtain a water-soluble konjac potato extract,
wherein the water-soluble konjac potato extract comprises sterylglucoside, with a content of fatty acid ester of equal to or less than 500 mg/100 g, a content of phytosterol of equal to or less than 5,000 mg/100 g, and a content of glucosylceramide of equal to or more than 5,000 mg/100 g, and
the first organic solvent and the second organic solvent are different from each other and from petroleum ether and chloroform.

5. The method according to claim 4, wherein the first organic solvent is ethanol.

6. The method according to claim 4, wherein the second organic solvent is acetone.
